# EUROPÄISCHE PATENTANMELDUNG

(11) **EP 4 400 120 A2**
(43) Veröffentlichungstag der Anmeldung: **17.07.2024**
(21) Anmeldenummer: 24179559.0
(22) Anmeldetag: 21.11.2020
(51) Int. Cl.: A61K 47/46

(54) **BAKTERIOPHAGEN-BEREITSTELLUNGEN SOWIE BAKTERIOPHAGEN-APPLIKATIONSVORRICHTUNG**

(30) Priorität: 14.01.2020 DE 102020100725
(62) Teilanmeldung aus: 20820325.7
(71) Anmelder: PHATEC GMBH, 24116 Kiel (DE)
(72) Erfinder: JUNGHANS, Simon Frank, 24116 Kiel (DE); GROSS, Justus, 24235 Lutterbek (DE)
(74) Vertreter: Simmons & Simmons LLP (Munich)

(57) **Zusammenfassung**

Die Erfindung betrifft Bakteriophagen-Bereitstellungen, nämlich intracorporale Bakteriophagen-Bereitstellung, naso-pharyngeale und pulmonale Bakteriophagen-Bereitstellung, cutane Bakteriophagen-Bereitstellung und Bakteriophagen-Nahtmaterial-Bereitstellung sowie weiter eine Zwei-Spritzen-Bakteriophagen-Bereitstellung, eine naso-pharyngeale und pulmonale Bakteriophagen-Bereitstellungs-Vorrichtung sowie eine Bakteriophagen-Sensitive-Tester-Applikation.

## Beschreibung

Die Erfindung betrifft Bakteriophagen-Bereitstellungen, nämlich intracorporale Bakteriophagen-Bereitstellung, naso-pharyngeale und pulmonale Bakteriophagen-Bereitstellung, cutane Bakteriophagen-Bereitstellung und Bakteriophagen-Nahtmaterial-Bereitstellung sowie weiter eine Zwei-Spritzen-Bakteriophagen-Bereitstellung, eine naso-pharyngeale und pulmonale Bakteriophagen-Bereitstellungs-Vorrichtung sowie eine Bakteriophagen-Sensitive-Tester-Applikation.

Als Bakteriophagen, kurz auch BPH oder Phagen, bezeichnet man verschiedene Gruppen von Viren, die auf Bakterien als Wirtszellen spezialisiert sind d.h. welche Bakterien spezifisch befallen. Damit wird der Wirt, beispielsweise ein Säugetier und insbesondere ein Mensch, nicht befallen. Eine Infektion von Bakterien oder anderen pathogenen Mikroorganismen durch virulente Phagen führt zum lytischen Zyklus und damit letztendlich zur Lyse und Zerstörung der Bakterien. Endotoxine sind Bakterientoxine, die im Gegensatz zu den Exotoxinen nicht von lebenden Bakterien ausgeschieden werden, sondern erst durch Autolyse freigesetzt werden.

Phagen haben in Medizin, Tiermedizin Biologie, Agrarwissenschaften, Lebensmittelverarbeitung, vor allem im Bereich der Gentechnologie, ein breites Anwendungsspektrum gefunden. So verwendet man Phagen in der Medizin aufgrund ihrer Wirtsspezifität zur Bestimmung von bakteriellen Erregern. Die Anwendung von Phagen zur Therapie bakterieller Infektionen entdeckte Felix d'Herelle lange vor Entdeckung des Penicillins und der Antibiotika. Später wurde die Phagentherapie jedoch mit der Einführung der Chemotherapie per Antibiotika als unpraktisch erachtet und geriet in Vergessenheit. Aufgrund der immer häufiger auftretenden multiplen Antibiotikaresistenzen wird derzeit wieder intensiv an der Anwendung von Bakteriophagen als Antibiotika-Ersatz in der Humanmedizin geforscht.

Eine Reihe von Publikation greifen diesen Stand der Technik auf und beschäftigen sich mit dem Stand der Phagentherapien allgemein und den regionalen Stati der medizinischen Zulassungen von Medikamenten, die auf Phagen basieren.

Aus der Druckschrift US 2004 / 0063 189 A1 ist eine Methode zur Identifikation und Bekämpfung des Bacillus Anthrax auf Oberflächen durch Phagen bekannt.

Weiter ist aus der Publikation Qadir et al. "Phage therapy: progress in pharmacokinetics", Braz. J. Pharm. Sei. 2018; 54(1):e17093, S.1-9 eine Ausführung zum allgemeinen Stand von Phagentherapien und den regionalen Stati der medizinischen Zulassungen von Medikamenten, die auf Phagen basieren bekannt.

Bakteriophagen können aus der Natur gewonnen werden. Dazu werden Wasserproben, Blutproben, Abstriche, menschliche oder tierische Sekrete, oder andere Proben genommen und auf Nährplatten ausgestrichen. Durch bebrüten dieser Platten (36 °C - 37 °C für 24 h) findet man, indiziert durch Lyse-Höfe, vorhandene Bakteriophagen. Durch Detektion der vorhandenen Prokaryoten kann eine erste Aussage getroffen werden, gegen welches Bakterium der gefundene Phage lytische aktiv ist.

Zur Aufreinigung wird der Plaque im Bakterienrasen ausgestochen und in einem Schnappdeckel-Gefäß mit flüssiger Nährlösung für mindestens 10 Minuten gevortext. Die flüssige Nährlösung wird entnommen, steril-filtriert und auf einer vorher mit dem entsprechenden Keim beimpften Nährmedium-Platte aufgetragen und wieder für 24 h bei 36 °C - 37 °C bebrütet. Eine Plaque wird wieder ausgestochen und wie beschrieben aufbereitet. Diesen Zyklus sollte man mindestens 5 Mal wiederholen, um sicher zu sein, dass die vorhandenen, isolierten Phagen nur Klone eines Phagen sind.

Zur Generierung größerer Mengen Phagenklone eines Phagen wird die steril filtrierte Phagenlösung aus dem Schnappdeckel-Gefäß nach mindestens 5-facher Aufreinigung auf einer beimpften Platte aufgebracht und erneut wie beschrieben bebrütet. Dann wird auf die Platte ein Extraktionspuffer gegeben, welcher auf dem Nährboden für 30 Minuten durch eine Schüttel-Apparatur bewegt wird. Der Extraktionspuffer wird entnommen und steril-filtriert, dabei erhält man eine sterile Phagen-Lösung.

Eine Bakteriophagen-Lösung kann über Zugabe von Stabilisatoren, wie bspw.: CaCl2 stabilisiert und über Substanzen zur pH-Einstellung wie bspw.: HCl, CH3COOH, CH3COO- physiologisch eingestellt werden. Der weitere Zusatz von Konservierungsmitteln kann dadurch indiziert sein, dass die Primärverpackung nicht vor dem Eindringen von Mikroorganismen schützt (ggf. bei nicht sterilen Produkten). Hier werden Konservierungsmittel wie bspw.: Kaliumsorbat eingesetzt.

Antimikrobielle Resistenzen entwickeln sich weltweit zum ernsten Problem für das Gesundheitswesen. Über Jahrzehnte hinweg wurde nur unzureichend an der Erforschung grundlegend neuer Antibiotika gearbeitet, folglich erreichten nur wenige Präparate den Markt. Der Druck ist seither enorm gewachsen, neue wirksame Konzepte zur Reduktion von Infektionen durch Problemerreger zu implementieren. Von politischer Seite wurde diese Dringlichkeit erkannt und umfangreiche Förderprogramme wurden sowohl national als auch international ins Leben gerufen. Eine tragende Säule vieler öffentlich finanzierter Maßnahmen ist die Erforschung und Entwicklung von Therapeutika, deren Wirkungen auf neuen Mechanismen beruhen und/oder die Bildung von Resistenzen minimieren.

In medizinischen Bereichen ist die Infektion eines Fremdkörpers mit einer erhöhten Komplikations- und Mortalitätsrate verbunden, diesbezüglich ist die derzeitige und prospektive Antibiotikum- Therapie ausgereizt.

Der dringende Bedarf an Antibiotika-Alternativen ist die Forderung für die diesseitige Erfindung.

Probleme ergeben sich durch die geringe Stabilität von Phagen im Körper, da sie in recht kurzer Zeit durch Fresszellen als Fremdkörper beseitigt werden.

Der vorliegenden Erfindung liegt die **Aufgabe** zugrunde, technische Bakteriophagen-Bereitstellungsverfahren sowie Bakteriophagen-Bereitstellungen, auch als Bakteriophagen-Depots bezeichnet, aufzuzeigen, die durch technische Vorrichtungen applizierbar sind sowie entsprechende Applikationsvorrichtungen hierzu aufzuzeigen, um entsprechende Bakteriophagen-Bereitstellungen zeitlich und örtlich dosierbar zu applizieren, um auf dieser Basis ein präventives Antibiotikum mit Breitbandwirkung ohne Nebenwirkungen einfach applizierbar bereitzustellen.

Insbesondere ist es Ziel, Bakteriophagen in unterschiedlichem Aggregatszustand und galenischer Zusammensetzung biologisch aktiv als Infektionsprophylaxe als Infektionstherapie an Fremdkörper und an homo- sowie xenogenes Gewebe zu applizieren.

Eine weitere abhängige Aufgabe ist es, eine Verbesserung der einzubringenden Bakteriophagen zu ermöglichen. Gelöst wird diese Unteraufgabe durch die kombinierte Applikation von Bakteriophagen und Endotoxinen.

**Gelöst** wird diese Aufgabe bzw. Aufgaben durch die erfindungsgemäßen Bakteriophagen-Bereitstellungen / -depots sowie Bakteriophagen-Bereitstellungen-Applikationsvorrichtung und Bakteriophagen-Bereitstellungen-Herstellungsverfahren gemäß Haupt- bzw. nebengeordneten Ansprüchen.

Die Intracorporale Bakteriophagen-Bereitstellung ist ausgebildet als steriles Bakteriophagen- Gel, wobei dieses freisetzungsmodulierbar ist, oder als sterile Bakteriophagen-Weichkapseln mit einem Gel oder als sterile Weichkapsel-Kette mit einem Bakteriophagen-Weichkapseln mit einem Gel auf einem monofilen, hydrolytisch abbaubaren Faden oder auf nicht abbaubarem Material, beispielsweise bei gewollter Dochtwirkung, oder als steriler Bakteriophagen-Schwamm, wobei der Schwamm mit einer Bakteriophagen Lösung oder einem Bakteriophagen-gel besprüht ist, oder ein Bakteriophagen-Gel durch Gefriertrocknen des Gels beispielsweise Lyophillisation, hergestellt ist.

Die Naso-pharyngeale und pulmonale Bakteriophagen-Bereitstellung ist ausgebildet als: Bakteriophagen-Lösung oder als Bakteriophagen-Pulver, wobei die zuvor genannten Bakteriophagen-Bereitstellungen über wenigstens eine der nachfolgenden Varianten vernebelbar sind, nämlich Verneblung über ein Beatmungsgerät mit Hilfe eines Phagen- Vernebelungs-Devices, Verneblung mittels Druckgas-Dosieraerosole, Verneblung über Düsenvernebler, Verneblung über Membranvernebler oder Verneblung über Pulverinhalator.

Die Cutane Bakteriophagen-Bereitstellung ist ausgebildet als steriles Bakteriophagen-Puder oder als steriler Bakteriophagen-Schwamm mit einem Bakteriophagen-Gel oder einem Bakteriophagen-Puder als Wundauflage oder als Schwamm in Form eines gefriergetrockneten Bakteriophagen-Gels.

Bakteriophagen-Nahtmaterial-Bereitstellung ist ein monofiles Nahtmaterial mit einer Bakteriophagen-Lösung oder mit einem Bakteriophagen-Gel zirkular besprüht ausgebildet ist oder es ist ein polyfiles Nahtmaterial mit einer Bakteriophagen-Lösung oder mit einem Bakteriophagen-Gel benetzt ist, wobei die Lösung oder das Gel am Fadenmaterial und/oder in den Kontaktzonen vorgesehen ist.

Die Zwei-Spritzen-Bakteriophagen-Bereitstellung kennzeichnet sich dadurch, dass eine erste Spritze mit einer Bakteriophagen-Lösung vorbereitet ist und eine zweite Spritze mit einem Gel vorbereitet ist, wobei diese zwei Spritzen miteinander, insbesondere über einen Konnektor, verbindbar sind, wobei eine Vermischung des Gels mit der Bakteriophagen-Lösung möglich ist und diese dann in einer Spritze zur Applikation vorliegt. Hierbei können die Gele und auch die Bakteriophagen-Lösungen entsprechend an die Bedürfnisse angepasst werden. Insbesondere können unterschiedliche Gele und/oder unterschiedliche Bakteriophagen-Lösungen zur unterschiedlichen Kombination miteinander vorgehalten und entsprechend miteinander zu einem individuellen Bakteriophagen-Lösung-Gel vermischt werden.

Die Naso-pharyngeale und pulmonale Bakteriophagen-Bereitstellungs-Vorrichtung, ist dadurch gekennzeichnet, dass eine Bakteriophagen-Lösung oder ein Bakteriophagen-Pulver mit wenigstens einer der nachfolgenden Vorrichtungen vernebelbar sind und die Bakteriophagen-Vernebelungsanordnung ausgebildet ist als (wenigstens eine Ausgestaltungsvariante der nachfolgenden Aufzählung):
- Beatmungsgerät mit einem Vernebelungs-Device oder einer Druckgas-Dosier-Aerosol-Kammer;
- Druckgas-Dosieraerosol-Applikator;
- Container-Inhalationsanordnung mit einer Vernebelungskammer und einem Düsenvernebler oder einem Membranvernebler;
- Inhalationsvorrichtung mit einem Druckgas-Dosieraerosol;
- Pulverinhalator.

Die Bakteriophagen-Sensitive-Tester-Applikation ist dadurch gekennzeichnet, dass in einem Behälter Bakteriophagen, eine bakterielle Nährlösung, sowie ein Farbstoff, angeordnet sind, wobei der Farbstoff mit bakteriellen Zellwänden interagieren kann.

Im Folgenden werden die zuvor genannten Ausführungen der Erfindung detailliert noch beschrieben:
Ein Bakteriophagen-Depot oder Phagen-Bereitstellung bzw. Depot ist eine Einheit, in der zumindest ein Bakteriophage oder Phage als eine Phagen-Applikation bereitgestellt wird, die die Stabilität nach Einbringung in einen Körper zeitlich definierbar aufrechterhält.

Es wurde insbesondere erkannt, dass der Bakteriophage sich sowohl für eine Behandlung als auch für eine Prophylaxe von bakteriellen Erkrankungen oder Entzündungen oder Pilzerkrankungen eignet, insbesondere aufgrund der sehr geringen damit verbundenen Nebenwirkungen. Aufgrund des Wirkmechanismus, der eine Vervielfältigung der aktiven Bakteriophagen erst bei Auftreten einer Infektion des Wirtes mit einem entsprechenden Bakterium oder Pilz beinhaltet, ist es möglich, prophylaktisch geringe Mengen an Bakteriophagen und Endotoxinen zu verabreichen, die den Wirtsorganismus nicht schädigen, die aber bei Auftreten von zu bekämpfenden Bakterien oder Pilzen in kurzer Zeit vervielfacht werden. Im Allgemeinen ist bei akuten Infektionen die Verabreichung hoher Dosen an Bakteriophagen möglich, da diese den pathogenen Mikroorganismus selektiv lysieren, ohne den Wirtsorganismus, z. B. einen Säuger, zu schädigen.

Bisher wurde davon ausgegangen, dass die immunstimulierende Wirkung von Endotoxinen für den Organismus eines Patienten nachteilige Folgen, wie etwa eine Temperaturerhöhung (Fieber) und zahlreiche weitere pathophysiologische Wirkungen hervorruft, wobei es bei Freisetzung hoher Endotoxindosen sogar zu einem irreversiblen Endotoxinschock kommen kann. Aus diesem Grund waren Endotoxine bisher in pharmazeutischen Zusammensetzungen, wie etwa zur Bekämpfung von Bakterien unerwünscht. Da davon ausgegangen wurde, dass Endotoxine für einen Heilungserfolg eher schädlich und zumindest unwichtig sind, wurden bisher zur Anwendung nur Endotoxin-freie Präparate in Betracht gezogen. Im Gegensatz dazu wurde nun überraschenderweise festgestellt, dass Endotoxine in Kombination mit Bakteriophagen eine positive Wirkung auf den Heilungserfolg haben.

Durch die kombinierte Applikation von Bakteriophagen und Endotoxinen ergibt sich eine synergetische Wirkung, insbesondere bei der Wundheilung. Dies wird darauf zurückgeführt, dass viele entzündliche Erkrankungen, wie beispielsweise der diabetische Fuß, auf drei Vorgängen basieren, nämlich einer Veränderung der Gefäße, einer Veränderung von Nervenleitungen sowie einer Infektion. Durch die Applikation von Bakteriophagen kann die Infektion bekämpft werden, wodurch der Gesamtheilungsprozess in Gang gesetzt wird. Die zusätzliche Gegenwart von Endotoxinen übt eine positive Wirkung auf den Gesamtheilungsprozess. Diese positive Wirkung beruht auf den immunstimulatorischen Eigenschaften von Endotoxinen.

Im Folgenden werden beispielhaft technische Umsetzungen der Bakteriophagen-Applikationen dargestellt. Die Auflistung orientiert sich an dem Weg der Applikation. Das Ziel aller dargestellten Bakteriophagen-Applikationen ist die Einbringung von therapeutisch aktiven Bakteriophagen zur Prävention und / oder Therapie und / oder Minimierung einer bakteriell bedingten Infektion, wobei es hier nicht um das therapeutische Verfahren geht, sondern vielmehr um die dazu notwendigen Produkte / Applikationen. Daneben werden Technologien dargestellt, welche die Bakteriophagen-Gewinnung, -Stabilisierung, oder die Testung der Sensitivität der Bakterien gegenüber vorhandenen Bakteriophagen behandeln.

### Variante A - Naso-pharyngeale und pulmonale Bakteriophagen-Applikation - Bakteriophagen-Lösung zur Vernebelung

Die pulmonale Bakteriophagen-Applikation kann über mehrere Arten erfolgen. Die drei Haupt-Möglichkeiten sind:
1. die Vernebelung von Bakteriophagen-Lösung(en) über das Beatmungsgerät mit Hilfe eines Phagen-Vernebelung-Devices
2. die Vernebelung von Bakteriophagen-Lösung(en) über
   a. Druckgas-Dosieraerosolen
   b. Vernebelung
      b1. Düsenvernebler
      b2. Membranvernebler
3. die pulmonale Applikation via Pulverinhalatoren
   - Möglichkeit 1:
      Die Bakteriophagen-Verneblung mit Hilfe des Beatmungsgerätes geschieht über ein Phagen-Vernebelung-Device, welches als Zwischenstück im Beatmungsschlauch mit diesem zu adaptieren ist und dem Beatmungsfilter nachgeschaltet sein muss. Durch ein Flatter-Ventil ist die Mitnahme der BPH während der Beatmung des Patienten gegeben. Dabei wird pro Beatmung, aufgrund des eingestellten Beatmungsdrucks, immer dieselbe Menge Bakteriophagen-Lösung appliziert. Das Flatter-Ventil schließt durch Druckumkehr bei der Ausatmung, sodass bei derselbigen keine Bakteriophagen-Lösung abgegeben wird. Zwischen BPH-Lösung und Flatter-ventil ist ein Mesh, welches die Tröpfchengröße der Vernebelung definiert. Für unterschiedliche Therapieziele und BPH-Target-Regionen können unterschiedliche Meshes angeboten, und damit unterschiedlich große Tröpfengrößen generiert werden.

Da das Phagen-Vernebelung-Device den Luftstrom nicht beeinträchtigt bleiben CO2- Messungen und auch die Verneblung weiterer Therapeutika weiterhin uneingeschränkt möglich.
- Möglichkeit 2:
   Die Bakteriophagen-Lösung wird mit einem entsprechenden Druckgas in ein mit einem Druckgas-Aerosol kompatiblem Container vorgehalten. Die Funktionsweise gleicht den allgemein bekannten Druckgas-Aerosolen.

Bei den anderen Vernebler-Formen ist die Bakteriophagen-Lösung als stabile Lösung als Einmaldosen abgepackt und vor dem Inhalieren dem entsprechenden Device zuzuführen.

Über alle diese Applikationsformen kann die therapeutische Dosis von Bakteriophagen - frei von der Umgebung - inhaliert werden. Die Lösung wird in Tröpfchengrößen < 5 Mikrometern vernebelt und gelangt so durch entsprechendes inhalieren in die Bronchialbäume bis zu den Alveolen.
- Möglichkeit 3:
   Die Bakteriophagen werden auf ein Trägermaterial, beispielsweise Lactose aufgebracht und als Bulk oder als Einmaldosis (Kapsel, Bilster, ...) für eine entsprechende Inhalation vorgehalten.

Die inhalativen Devices funktionieren gleich denen auf dem Markt befindlichen Pulver inhalatoren:
Durch einen Mechanismus des Devices wird die Kapsel, der Blister oder dergleichen angestochen und das Pulver damit freigegeben. Durch kräftiges Einatmen des Patienten wird das Pulver eingeatmet und zuvor im Device über Obstacles so verwirbelt, dass sie dem Luftstrom folgen.

### - Sprühtrocknung

In der Bakteriophagen-Lösung wird ein geeignetes Trägermaterial, beispielsweise Lactose, gelöst. Die Lösung wird mittels Sprühtrocknung wieder in den festen Aggregatzustand überführt. Dabei resultiert ein fester, amorpher Zustand des Trägermaterials. Dieser Zustand generiert ein sofortiges Lösen des Materials durch die extrazelluläre Flüssigkeit und eine damit einhergehende Freisetzung der Bakteriophagen. - Aufsprühen der Bakteriophagen auf das entsprechende Trägermaterial Als zweite, weitere Möglichkeit des Aufbringens der entsprechen Bakteriophagen -Lösung auf das jeweilige Trägermaterial gilt das Aufsprühen, dabei wird das Trägermaterial unter Düsen, durch welche die Bakteriophagen-Lösung aufgesprüht wird, bewegt und transportiert. Es ist dabei wichtig, dass sich das Trägermaterial bewegt um eine Benetzung von allen Seiten zu generieren.

### Variante B - intracorporale Applikation:

### 1) Steriles Bakteriophagen-Gel (freisetzungsmoduliert)

Das sterile Bakteriophagen-Gel ist überall intracorporal applizierbar. Die Herstellung erfolgt aus der entsprechenden Bakteriophagen-Lösung heraus:
Ein Gel wird mittels eines Gelbildners (bspw.: HPMC, ...), ohne den Wasseranteil, den später die Bakteriophagen-Lösung stellt, hergestellt und sterilisiert. Es können adhäsive Substanzen zugemischt werden, die die Adhäsion an unterschiedlichen Materialen (PTFE, Keramik, Dacron, Titan, ...) verbessern. Die Bakteriophagen-Lösung wird unter sterilen Bedingungen steril-filtriert und in einer Spritze steril vorgehalten. Das sterilisierte Gel wird unter sterilen Bedingungen auch in einer Spritze vorgehalten. Zur besseren Lagerung werden die beiden Komponenten durch eine Zwei-Spritzen-Technik durch ein operierendes Team erst vor Applikation vermischt.

Die Eigenschaften des resultierenden Gels und damit auch die Bakteriophagen- Freisetzungsrate aus dem selbigen werden über die benutze Menge an Gelbildner definiert.

Aus diesem Grund sollen unterschiedliche Gelgrundlagen in Spritzen vorgehalten werden. Alle Bakteriophagen-Lösungen, welche sich in ihrer Zusammensetzung der Bakteriophagen unterscheiden, sind mit allen Gelgrundlagen durch die Zwei-Spritzen-Technik kombinierbar.

Ein Operateur kann also intra-operativ entscheiden, welche Viskosität / Freisetzung und welche Bakteriophagen-Zusammensetzung er applizieren möchte. Dabei sind weniger viskose Gele schnell freisetzend und hoch-visköse Bakteriophagen-Gele setzen über einen längeren Zeitraum frei. Die einzelnen Spritzen (Gelgrundlage und Bakteriophagen-Lösung) sind jeweils steril verpackt und werden auf Verlangen vom unsterilen OP-Personal steril angereicht. Das sterile OP-Personal mischt die Komponenten mit Hilfe der Zwei-Spritzen-Technik über einen vorgegebenen Zeitraum. Das Bakteriophagen-Gel ist jetzt zur Applikation bereit.

Weitere Herstellungsvarianten:
- über maschinelle Produktion werden Bakteriophagen-Lösungen mit einem ersten Teil des Gels vermischt und steril-filtriert. Die Modulation dieses Grundgels erfolgt durch entsprechendes Zumischen des Gelbildners unter sterilen Bedingungen in einem nachgeschalteten Produktionsschritt;
- maschinelle, sterile Abfüllung von Bakteriophagen-Lösung und Grundgel. Das Grundgel wird anschließend im Endbehältnis sterilisiert. Ein Zusammenbringen ist flexibel gleich dem oben, initial, beschriebenen, händischen Verfahren.

Weitere Möglichkeiten steriler Bakteriophagen-Gele:
A. die Bakteriophagen sind in einer Hydrogel-Matrix homogen verteilt eingebettet. Die Freisetzung aus dem Hydrogel wird durch den Anteil an Hydrogelbildner im Verhältnis zum Wasseranteil bestimmt. Das Produkt interagiert nicht mit Haut- oder Schleimhautzellen, ist gegen den menschlichen Organismus inert und kann intra-, sowie extra-corporal zum Einsatz kommen; mögliche Gelbildner sind Carbomere, Celluloseether, Gelatine, Alginate, Betonid, hochdisperses Siliciumdioxid.
B. Die Phagen sind in einer lipophilen Gel-Matrix eingebettet. Die Freisetzung aus dem Hydrogel wird durch den Anteil an Lipophilgelbildner im Verhältnis zum Wasseranteil bestimmt. Das Produkt ist über den antibakteriellen Mechanismus hinaus stark rückfettend und unterstützt den natürlichen Wundheilungsprozess als Ergänzung zur antibakteriellen Therapie / Prävention. Es kann insbesondere extra-corporal, besonders cutan, angewandt werden;
   Mögliche lipophile Gelbildner sind hochdisperses Siliciumdioxid, Aluminium seifen, Zinkseifen, insbesondere jeweils mit entsprechender, lipophiler Grundlage, wie Mineralöl oder flüssige Triglyceride.
C. Die Bakteriophagen sind in einer amphiphilen Gel-Matrix eingebettet. Die Freisetzung aus dem Hydrogel wird durch den Anteil an amphiphilem Gelbildner im Verhältnis zum Wasseranteil bestimmt. Das Produkt kann extra-corporate Anwendung finden (vgl. "B."). Besonders dient es der weiteren Verarbeitung.

Alle Gele können auch über die Spritze minimalinvasiv eingebracht werden. Auch eine percutane Applikation, bspw.: in einen Abszess, ist also möglich.

### 2) sterile Bakteriophagen-Weichkapseln

Zuerst wird ein Gel wie oben beschrieben hergestellt. Der Unterschied ist hier, dass das Endprodukt, also das "Endgel" hergestellt wird. Dann wird dieses Gel, welches in Viskosität, BPH-Gehalt und -Zusammensetzung frei variieren kann, steril in Weichkapseln mit Hilfe des Rotary-Die-Verfahrens, des Tropf-Verfahrens oder anderen geeigneten technischen Verfahren zur Generierung von Weichkapseln, abgefüllt. Als Kapselmaterial wird beispielsweise Gelatine genommen. Das Kapselmaterial wird nach Anforderungen ausgewählt. Entsprechende Anforderungen können die Schnelligkeit der Freisetzung und die Weite der Therapie sein. Dabei ist gemeint, dass weniger viskose Formen durch Körperwasser und Körpertemperatur schneller und stärker verflüssigen und dadurch stärker vom Applikationsort abfließen, wodurch ein größeres Areal abgedeckt wird. Soll die BPH-Formulierung eher am Ort der Applikation gehalten werden, sollte eine höher viskose Form genommen werden, die durch Körpertemperatur und Körperflüssigkeit weniger schnell verflüssigt wird und länger am Applikationsort verbleibt. Dabei kann durch den Prozess ferner die Dicke der Kapselhülle und die Größe der Kapsel selber variiert werden.

### 3) sterile Weichkapsel-Kette

Wie oben beschrieben werden Weichkapseln nach entsprechenden Anforderungen hergestellt. Nach entsprechender Herstellung werden die Kapseln mit fixem Abstand zueinander auf einen monofilen, hydrolytisch abbaubaren Faden aufgezogen. Dies erlaubt eine intra-operative Applikation, auch in Körperhöhlen. Der Aufzug der Kapseln auf ein ein nicht-abbaubares Material dient der Applikation mit beispielsweise gewollter Dochtwirkung.

### 4) sterilier Bakteriophagen-Schwamm

Als Grundlage dienen die bereits vorgestellten Gele. Diese werden unter genauer Einstellung und Kontrolle der einflussnehmenden Parameter, sowie unter sterilen Bedingungen, gefriergetrocknet (= lyophillisiert). Weiter sind alle Trocknungsmöglichkeiten zur Herstellung einer festen Bakteriophagen-Applikation aus den entsprechenden Gelen möglich.
a. Die Phagen sind nach Gefriertrocknung in einer festen Matrix eingebettet. Die Freisetzung passiert radiär -von außen nach innen- und entspricht einem retardierten Mechanismus. Die Freisetzung wird durch den Anteil an Gelbildner im Verhältnis zum Wasseranteil bestimmt.
b. Die Bakteriophagen sind in einer nun festen lipophilen Gel-Matrix eingebettet. Die Freisetzung passiert radiär-von außen nach innen- und entspricht einem retardierten Mechanismus. Sie wird durch den Anteil an Gelbildner im Verhältnis zum Wasseranteil bestimmt.
c. Die Bakteriophagen sind in einer nun festen amphiphilen Gel-Matrix eingebettet. Die Freisetzung passiert radiär-von außen nach innen- und entspricht einem retardierten Mechanismus. Sie wird durch den Anteil an Gelbildner im Verhältnis zum Wasseranteil bestimmt.

Diese Galenik führt zu einer retardierten Freisetzung des antibiotischen Agens. Die Freisetzungsrate wird durch das Verhältnis Oberfläche zu Volumen bestimmt. Des Weiteren können dem Ausgangs-Gel Kollagen-Fasern zugemischt werden, die die entsprechende Gelmatrix als Stabilisator unterstützen.

### Variante C - cutane Bakteriophagen-Applikation / Wundauflage

### - steriles Bakteriophagen-"Puder"

Es wird ein Bakteriophagen-Produkt wie das Bakteriophagen-Pulver zur Inhalation steril hergestellt und in einer dicht verschlossenen, vor Feuchtigkeit geschützten Umverpackung vorgehalten. Dieses Produkt ist besonders für nässende Wunden wie bspw. Brandwunden geeignet.

### - steriler Bakteriophagen-Schwamm als Wundauflage

Entsprechend dem zuvor erläuterten Bakteriophagen-Schwamm. Dieser wird mit einer Klebematrix / einem Klebelayer überzogen, dessen Maße die Maße des Schwammes übertrifft. Diese Kleberänder die beispielsweise aus Poly-Acrylamid als Klebekomponente bestehen, dienen als Adaptionsmechanismus auf der gesunden Haut.

### Variante D - Bakteriophagen-Nahtmaterial

Bei der Nahtherstellung z.B. kann dabei monofiles Nahmaterial nach erfolgter Produktion noch einmal auf eine Temperatur von maximal 35 °C erwärmt werden, damit sich das Nahtmaterial lockert und unter weiterer Anwendung der Wärme die Naht mit einer Bakteriophagen-Lösung oder mit einem Bakteriophagen-Gel zirkulär besprüht wird und anschließend beim Abkühlprozess die Bakteriophagen-Lösung aufgenommen ist.

Bei polyfilem Nahtmaterial, d.h. Nahtmaterial bestehend aus mehr als einem Filament, erfolgt keine Erwärmung, sondern lediglich eine Besprühung mit Bakteriophagen-Lösung oder mit einem Bakteriophagen-Gel, da die Lösung bzw. das Gel sich in die Kontaktzonen der einzelnen Filamente setzt und unter anschließender Kälte-Einwirkung am Fadenmaterial verbleibt.

Jedes Nahtmaterial kann, unabhängig von der Produktion, auch in das entsprechende Gel eingelegt und verpackt werden, wobei das Gel so während der gesamten Lagerung den Faden umschließt.

Besonders bei hydrolytisch spaltbarem Nahtmaterial ist insbesondere darauf zu achten, dass das Gel einen lipophilen Gelbildner hat und die Phagen zur Gelgenerierung in einer W/O- Emulsion vorliegen. Die Bakteriophagen-Lösung stellt dabei das vom Öl eingeschlossene Wasser. Diese W/O-Bildung kann mit Hilfe von Mizellen oder Emulgatoren erfolgen.

Im Folgenden werden beispielhaft technische Umsetzungen der Bakteriophagen-Applikationen dargestellt. Die Auflistung orientiert sich an dem Weg der Applikation. Das Ziel aller dargestellten Bakteriophagen-Applikationen ist die Einbringung von therapeutisch aktiven Bakteriophagen zur Prävention und / oder Minimierung einer bakteriell bedingten Infektion. Daneben werden Technologien dargestellt, welche die Bakteriophagen- Gewinnung, -Stabilisierung oder die Testung der Sensitivität der Bakterien gegenüber vorhandenen Bakteriophagen behandeln.

Es werden Ausführungsbeispiele der Erfindung anhand der beiliegenden Zeichnungen in der **Figurenbeschreibung** detailliert beschrieben und zusätzliche nachfolgend auch weitere andere Ausführungsvarianten aufgezeigt, die nicht figürlich dargestellt sind. Diese Ausführungen sollen die Erfindung erläutern und sollen nicht beschränkend zu werten sein:

Es zeigen:
- Fig. 1: eine schematische Darstellung eines Ausführungsbeispiels einer ersten Applikation für Bakteriophagen;
- Fig. 2: eine schematische Darstellung eines Ausführungsbeispiels einer zweite pulmonalen Bakteriophagen-Applikations-Vorrichtung;
- Fig. 3: eine entsprechend mit Bakteriophagen und Trägermaterial gefüllte und für entsprechend funktionierende Devices ausgebildete Hartkapsel;
- Fig. 4: eine weitere Applikationsvariantenart, wobei hier eine intracorporale Bakteriophagen-Applikation gezeigt wird;
- Fig. 5: eine weitere Applikation, wobei eine sterile Bakteriophagen-Weichkapsel-Applikation dargestellt ist;
- Fig. 6: eine Bakteriophagen-Weichkapsel-Kette-Applikation;
- Fig. 7: eine Bakteriophagen-Schwamm-Gel-Applikationsvariante;
- Fig. 8: eine schematische Darstellung eines Ausführungsbeispiels einer Bakteriophagen-Sensitive-Tester-Applikation in der Ausgangssituation und
- Fig. 9: eine schematische Darstellung eines Ausführungsbeispiels der Bakteriophagen-Sensitive-Tester-Applikation aus Fig. 9 in der Testphase.

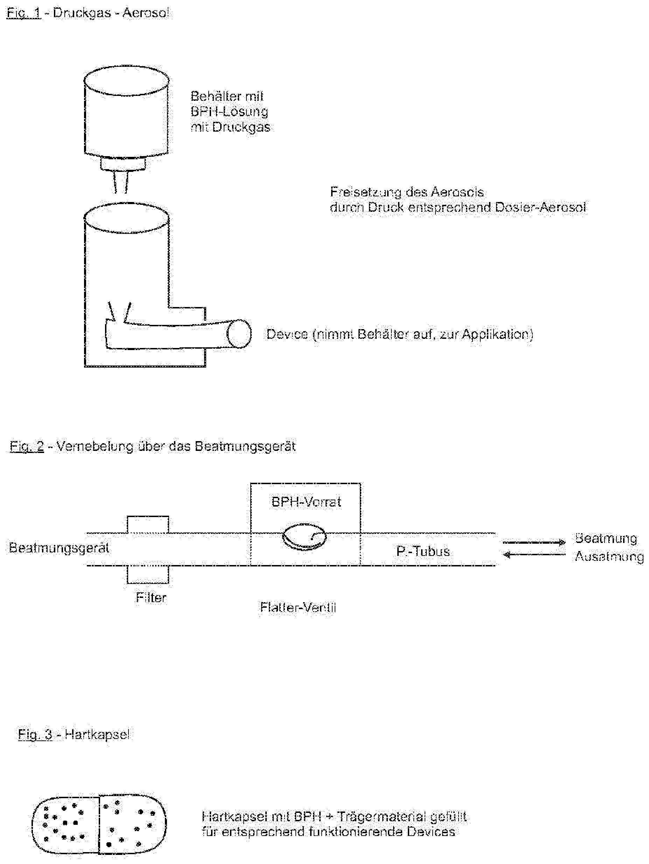

In **Fig. 1** ist eine schematische Darstellung eines Ausführungsbeispiels einer ersten Applikation dargestellt, wobei hier eine pulmonale Applikation die Lunge und Atemwege betreffende Bakteriophagen-Applikation gezeigt ist, wobei eine Bakteriophagen-Lösung zur Vernebelung über pulmonale Bakteriophagen-Applikations-Vorrichtungen erfolgt.

Die pulmonale Bakteriophagen-Applikation erfolgt dabei über mehrere Arten von pulmonalen Bakteriophagen-Applikations-Vorrichtungen, die an den Atemweg eines Nutzers direkt herangeführt werden oder an eine bestehende Atemversorgung ankoppelbar sind.

Eine erste pulmonale Bakteriophagen-Applikations-Vorrichtung weist eine Druckgas-DosierAerosol Kammer auf über die Bakteriophagen-Lösung(en) an die Beatmungsvorrichtung koppelbar sind. Die Bakteriophagen-Lösung wird mit einem entsprechenden Druckgas in ein mit einem Druckgas-Aerosol kompatiblem Container vorgehalten.

**Fig. 2** zeigt eine zweite pulmonale Bakteriophagen-Applikations-Vorrichtung. Diese weist eine Vernebelungs-Kammer auf, über die Bakteriophagen-Lösung(en) an die Beatmungsvorrichtung koppelbar sind, wobei die Vernebelungskammer Düsenvernebler und/oder Membranvernebler aufweist.

Die Bakteriophagen-Verneblung mit Hilfe des Beatmungsgerätes geschieht über ein Phagen-Vernebelungs-Device, welches als Zwischenstück im Beatmungsschlauch mit diesem zu adaptieren ist und dem Beatmungsfilter nachgeschaltet sein muss. Durch ein Flatter-Ventil ist die Mitnahme der Bakteriophagen während der Beatmung des Patienten gegeben. Dabei wird pro Beatmung, aufgrund des eingestellten Beatmungsdrucks, immer dieselbe Menge BPH- Lösung appliziert. Das Flatter-Ventil schließt durch Druckumkehr bei der Ausatmung, sodass bei derselbigen keine Bakteriophagen-Lösung abgegeben wird. Zwischen BPH-Lösung und Flatter-Ventil ist ein Mesh, welches die Tröpfchengröße der Vernebelung definiert. Für unterschiedliche Therapieziele und Bakteriophagen-Target-Regionen können unterschiedliche Meshes angeboten, und damit unterschiedlich große Tropfengrößen generiert werden. Da das Phagen-Vernebelung-Device den Luftstrom nicht beeinträchtigt bleiben C02-Messungen und auch die Verneblung weiterer Therapeutika weiterhin uneingeschränkt möglich. Die Bakteriophagen-Lösung wird bevorzugt als stabile Lösung als Einmaldosen abgepackt und vor dem Inhalieren dem entsprechenden Device zuzuführen.

Eine weitere (nicht figürlich dargestellt) pulmonale Bakteriophagen-Applikationsvorrichtung weist einen Pulverinhalator auf. Die Bakteriophagen werden dabei auf ein Trägermaterial aufgebracht und als Bulk oder als Einmaldosis (Kapsel, Blister, oder dgl.) für eine entsprechende Inhalation vorgehalten. Der inhalative Pulver-Inhalator nutzt eine Sprühtrocknung. Wobei in der Bakteriophagen-Lösung ein geeignetes Trägermaterial, beispielsweise Lactose, gelöst wird. Die Lösung wird mittels Sprühtrocknung wieder in den festen Aggregatzustand überführt. Dabei resultiert ein fester, amorpher Zustand des Trägermaterials. Dieser Zustand generiert ein sofortiges Lösen des Materials durch die extrazelluläre Flüssigkeit und eine damit einhergehende Freisetzung der Bakteriophagen. Eine weitere Möglichkeit des Aufbringens der entsprechen Bakteriophagen-Lösung auf das jeweilige Trägermaterial erfolgt über das Aufsprühverfahren, wobei das Tägermaterial unter Düsen transportiert wird, durch welche die Bakteriophagen-Lösung aufgesprüht wird. Es ist dabei besonders vorteilhaft, wenn sich das Trägermaterial bewegt, um eine Benetzung von allen Seiten zu generieren.

Über alle diese pulmonalen Bakteriophagen-Applikationen kann die therapeutische Dosis von Bakteriophagen - frei von der Umgebung - inhaliert werden. Die Lösung wird insbesondere in Tröpfchengrößen kleiner 5 Mikrometern vernebelt und gelangt so durch entsprechendes Inhalieren in die Bronchialbäume bis zu den Alveolen.

**Fig. 3** zeigt eine entsprechend mit Bakteriophagen und Trägermaterial gefüllte und für entsprechend funktionierende Devices ausgebildete Hartkapsel.

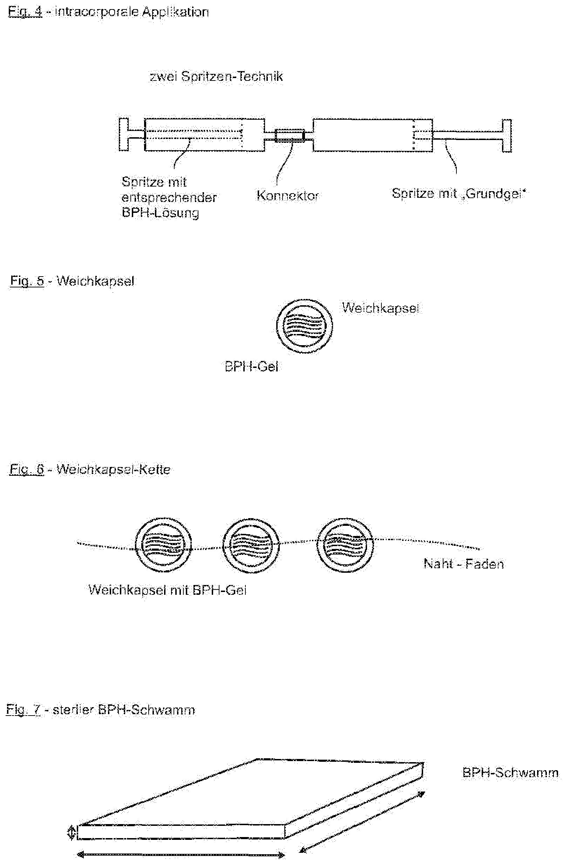

**Fig. 4** zeigt eine weitere Applikationsvariantenart, wobei hier eine intracorporale Bakteriophagen-Applikation gezeigt wird, wobei aus einer Bakteriophagen-Lösung ein steriles Bakteriophagen-Gel hergestellt wird und über Bakteriophagen-Applikations-Vorrichtungen überall intracorporal applizierbar ist.

Bei der Herstellung wird ein Gel mittels eines Gelbildners, beispielsweise HPMC oder dgl. , ohne den Wasseranteil der späteren Bakteriophagen-Lösung, hergestellt und sterilisiert. Es können dabei adhäsive Substanzen zugemischt werden, die die Adhäsion an unterschiedlichen Materialen (PTFE, Keramik, Dacron, Titan, Zinkoxid...) verbessern.

Die Bakteriophagen-Lösung wird unter sterilen Bedingungen steril-filtriert und z.B. in einer Spritze steril vorgehalten. Das sterilisierte Gel wird unter sterilen Bedingungen z.B. in einer Spritze vorgehalten. Zur besseren Lagerung werden die beiden Komponenten durch eine Zwei- Spritzen-Technik zeitlich erst unmittelbar vor einer Applikation vermischt.

Die Eigenschaften des resultierenden Gels und damit auch die Bakteriophagen- Freisetzungsrate aus dem selbigen werden über die benutze Menge an Gelbildner definiert. Aus diesem Grund werden unterschiedliche Gelgrundlagen z.B. in Spritzen vorgehalten. Alle Bakteriophagen-Lösungen, welche sich in ihrer Zusammensetzung der Bakteriophagen unterscheiden, sind mit allen Gelgrundlagen damit durch die Zwei-Spritzen-Technik kombinierbar.

Weitere Möglichkeiten der Bereitstellung sind z.B. eine schrittweise Vorgehensweise bei der z.B. über maschinelle Produktion Bakteriophagen-Lösungen mit einem ersten Teil des Gels vermischt und steril-filtriert werden und die Modulation dieses Grundgels durch entsprechenden Zumischen des Gelbildners unter sterilen Bedingungen in einem nachgeschalteten Produktionsschritt erfolgt.

Weiter ist es möglich, die maschinelle, sterile Abfüllung von BPH-Lösung und Grundgel durchzuführen und das Grundgel anschließend im Endbehältnis zu sterilisieren. Ein Zusammenbringen kann dann flexibel erfolgen.

Weitere Möglichkeiten sterile Bakteriophagen-Gele bereitzustellen sind z.B. die Phagen in einer Hydrogel-Matrix homogen verteilt einzubetten. Die Freisetzung aus dem Hydrogel wird dabei durch den Anteil an Hydrogelbildner im Verhältnis zum Wasseranteil bestimmt. Das Produkt interagiert nicht mit Haut- oder Schleimhautzellen, ist gegen den menschlichen Organismus inert und kann intra-, sowie extra-corporal zum Einsatz kommen, oder die Phagen in einer lipophilen Gel-Matrix einzubetten. Die Freisetzung aus dem Hydrogel wird dabei durch den Anteil an Lipophilgelbildner im Verhältnis zum Wasseranteil bestimmt. Das Produkt ist über den antibakteriellen Mechanismus hinaus stark rückfettend und unterstützt den natürlichen Wundheilungsprozess als Ergänzung zur antibakteriellen Therapie / Prävention. Es kann extra-corporal, insbesondere cutan angewandt, oder die Phagen in einer amphiphilen Gel-Matrix einzubetten. Die Freisetzung aus dem Hydrogel wird dabei durch den Anteil an amphiphilem Gelbildners im Verhältnis zum Wasseranteil bestimmt. Das Produkt kann zudem auch extracorporale Anwendung finden.

Alle Gele können auch über die Spritze minimalinvasiv eingebracht werden. Auch eine percutane Applikation, bspw. in einen Abszess, ist nunmehr erstmalig möglich, um diesen zu behandeln.

Es kann unmittelbar vor der Applikation die Viskosität, Freisetzungsrate und BPH-Zusammensetzung eingestellt werden. Dabei sind weniger viskose Gele schnell freisetzend und hoch-visköse BPH-Gele setzen über einen längeren Zeitraum frei.

**Fig. 5** zeigt eine weitere Applikation, wobei eine sterile Bakteriophagen-Weichkapsel- Applikation dargestellt ist, wobei zuerst ein Gel hergestellt wird, welches im Unterschied zum zuvor beschriebenen Gel bereits das Endprodukt als Gel stellt, also keine weiteren Produktionsschritte aufweist. Dann wird dieses Gel, welches in Viskosität, Bakteriophagen- Gehalt und - Zusammensetzung frei variieren kann, steril in Weichkapseln mit Hilfe des Rotary- Die-Verfahrens, des Tropf-Verfahrens oder anderen geeigneten technischen Verfahren zur Generierung von Weichkapseln abgefüllt. Als Kapselmaterial wird beispielsweise Gelatine genommen. Das Kapselmaterial wird nach Anforderungen ausgewählt. Entsprechende Anforderungen können die Schnelligkeit der Freisetzung und die Weite der Therapie sein.

Dabei ist gemeint, dass weniger viskose Formen z.B. durch Körperwasser und Körpertemperatur schneller und stärker verflüssigen und dadurch stärker vom Applikationsort abfließen, wodurch ein größeres Areal abgedeckt wird. Soll die BPH-Formulierung eher am Ort der Applikation gehalten werden, sollte eine höher viskose Form genommen werden, die durch Körpertemperatur und Körperflüssigkeit weniger schnell verflüssigt wird und länger am Applikationsort verbleibt.

Dabei kann durch den Prozess ferner die Dicke der Kapselhülle und die Größe der Kapsel selber variiert werden.

Die Bakteriophagen-Weichkapsel-Applikation stellt damit ein Bakteriophagen-Depot oder Phagen-Depot als eine Einheit bereit in der zumindest eine Bakteriophage oder Phage als eine Phagen-Applikation bereitgestellt wird die die Stabilität nach Einbringung in einen Körper zeitlich definierbar aufrecht erhält.

**Fig. 6** zeigt ergänzend zu den vorherigen Ausführungen eine weitere Applikation, wobei hier eine sterile Bakteriophagen-Weichkapsel-Kette-Applikation vorliegt. Wie bei der Bakteriophagen-Weichkapsel-Applikation werden Weichkapseln nach entsprechenden Anforderungen hergestellt.

Nach entsprechender Herstellung werden die Kapseln mit fixem Abstand zueinander insbesondere auf einen monofilen, hydrolytisch abbaubaren Faden aufgezogen. Dies erlaubt eine intra-operative Applikation, auch in Körperhöhlen.

**Fig. 7** zeigt eine Bakteriophagen-Schwamm-Gel-Applikationsvariante. Eine weitere Applikation zeigt eine sterile Bakteriophagen-Schwamm-Gel-Applikation, wobei als Grundlage die bereits vorgestellten Gele dienen. Diese werden unter genauer Einstellung und Kontrolle der einflussnehmenden Parameter, sowie unter sterilen Bedingungen, gefriergetrocknet/ lyophillisiert. Weiter sind alle Trocknungsmöglichkeiten zur Herstellung einer festen Bakteriophagen-Applikation aus den entsprechenden Gelen möglich.

Die Phagen sind nach Gefriertrocknung in einer festen Matrix eingebettet, in einer nun festen lipophilen Gel-Matrix eingebettet oder in einer nun festen lipophilen Gel-Matrix eingebettet als Bakteriophagen-Schwamm-Gel-Applikation bereitstellbar. Wobei die Freisetzung jeweils radiär-von außen nach innen- erfolgt. Dies entspricht einem retardierten Mechanismus. Die Freisetzung wird dabei durch den Anteil an Gelbildner im Verhältnis zum Wasseranteil bestimmt.

Diese Galenik führt zu einer retardierten Freisetzung des antibiotischen Agens. Die Freisetzungsrate wird durch das Verhältnis Oberfläche zu Volumen bestimmt. Des Weiteren können dem Ausgangs-Gel Kollagen-Fasern zugemischt werden, die die entsprechende Gelmatrix als Stabilisator unterstützen.

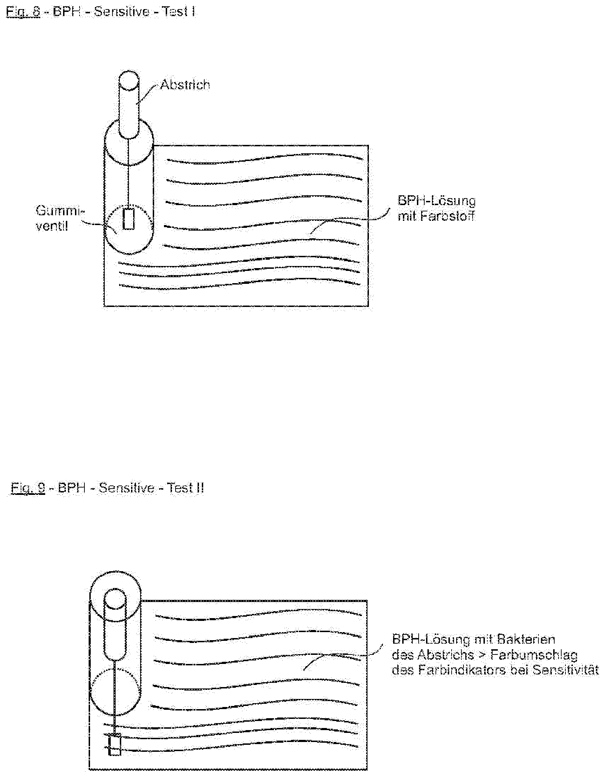

**Fig. 8** und **Fig. 9** zeigen eine Bakteriophagen-Sensitive-Tester-Applikation, wobei in einem Behälter entsprechende Bakteriophagen, eine bakterielle Nährlösung (flüssig), sowie ein Farbstoff, welcher mit bakteriellen Zellwänden interagiert, vorgehalten werden. Innerhalb der Interaktion ist der Farbstoff A. sichtbar / B. farblos. Eine Probe, z.B. ein Abstrich (Probennehmer im Set enthalten) wird als Testprobe direkt in den Behälter gegeben. Der Farbstoff interagiert mit den Bakterien und ist A. sichtbar / B. nicht sichtbar. Der Tester wird für 12 h bis 24 h bei 36°C bebrütet. Nach dieser Zeit ist die Sensitivität der Bakterien gegenüber der vorgelegten BPH dadurch angezeigt, dass die Lösung A. weniger intensiv gefärbt ist / B. gefärbt ist.

### Weitere Applikationen:

Diese Aufzählung von Bakteriophagen-Applikationen ist nicht abschließend. Auch können Kombinationen der Bakteriophagen-Applikationen bereitgestellt werden und Materialien mit den Bakteriophagen-Applikationen vor einer Verwendung überzogen werden und damit weitere Applikationen generieren. Ein besonders hervorzuhebendes Anwendungsbeispiel für eine gezielte Applikation von Phagen ist die Prothetik.

Protheseninfektionen stellen auch heute noch eine der gravierendsten Komplikationen der rekonstruktiven Chirurgie dar. Protheseninfektion, insbesondere wenn beispielsweise die Aorta betroffen ist, haben oft einen letalen Verlauf; gerade dieses chirurgische Fachgebiet stellt sich nicht nur als hochkompliziert, sondern auch aufgrund der hohen Anzahl verwendeter Kunststoffprothesen als besonders komplikationsträchtig dar.

Weitere Ausführungsformen:
1. Intracorporale Bakteriophagen-Bereitstellung,
   **dadurch gekennzeichnet, dass**
   die Bakteriophagen-Bereitstellung ausgebildet ist als:
   - steriles Bakteriophagen-Gel, wobei dieses freisetzungsmodulierbar ist;
      oder
   - sterile Bakteriophagen-Weichkapseln mit einem Gel;
      oder
   - sterile Weichkapsel-Kette mit einem Bakteriophagen-Weichkapseln mit einem Gel auf einem monofilen, hydrolytisch abbaubaren Faden oder auf einem nicht-abbaubaren Material;
      oder
   - steriler Bakteriophagen-Schwamm, wobei der Schwamm mit einer Bakteriophagen Lösung oder einem Bakteriophagen-Gel besprüht ist oder ein Bakteriophagen-Gel gefriergetrocknet ist.
2. Naso-pharyngeale und pulmonale Bakteriophagen-Bereitstellung,
   **dadurch gekennzeichnet, dass**
   die Bakteriophagen-Bereitstellung ausgebildet ist als:
   Bakteriophagen-Lösung oder als Bakteriophagen-Pulver,
   wobei die zuvor genannten Bakteriophagen-Bereitstellungen über wenigstens eine der nachfolgenden Varianten vernebelbar sind:
      - Verneblung über ein Beatmungsgerät mit Hilfe eines Phagen-Vernebelungs-Devices;
      - Verneblung mittels Druckgas-Dosieraerosole;
      - Verneblung über Düsenvernebler;
      - Verneblung über Membranvernebler;
      - Verneblung über Pulverinhalator.
3. Cutane Bakteriophagen-Bereitstellung,
   **dadurch gekennzeichnet, dass**
   die Bakteriophagen-Bereitstellung ausgebildet ist als:
   - steriles Bakteriophagen-Puder
      oder
   - steriler Bakteriophagen-Schwamm mit einem Bakteriophagen-Gel oder einem Bakteriophagen-Puder als Wundauflage.
4. Bakteriophagen-Nahtmaterial-Bereitstellung,
   **dadurch gekennzeichnet, dass**
   - ein monofiles Nachtmaterial mit einer Bakteriophagen-Lösung oder mit einem Bakteriophagen-Gel zirkular besprüht ausgebildet ist
      oder
   - ein polyfiles Nahtmaterial mit einer Bakteriophagen-Lösung oder mit einem Bakteriophagen-Gel benetzt ist, wobei die Lösung oder das Gel am Fadenmaterial und/oder in den Kontaktzonen vorgesehen ist.
5. Zwei-Spritzen-Bakteriophagen-Bereitstellung,
   **dadurch gekennzeichnet, dass**
   eine erste Spritze mit einer Bakteriophagen-Lösung vorbereitet ist und eine zweite Spritze mit einem Gel vorbereitet ist, wobei diese zwei Spritzen miteinander verbindbar sind, wobei eine Vermischung des Gels mit der Bakteriophagen-Lösung möglich ist und diese dann in einer Spritze zur Applikation vorliegt.
6. Zwei-Spritzen-Bakteriophagen-Bereitstellung nach der vorangegangenen Ausführungsform, **dadurch gekennzeichnet, dass**
   das unterschiedliche Gele und/oder unterschiedliche Bakteriophagen-Lösungen zur unterschiedlichen Kombination miteinander vorhaltbar sind und entsprechend miteinander zu einem individuellen Bakteriophagen-Lösung-Gel mischbar sind.
7. Naso-pharyngeale und pulmonale Bakteriophagen-Bereitstellungs-Vorrichtung, **dadurch gekennzeichnet, dass**
   eine Bakteriophagen-Lösung oder ein Bakteriophagen-Pulver mit wenigstens einer der nachfolgenden Vorrichtungen vernebelbar sind und die Bakteriophagen-Vernebelungsanordnung ausgebildet ist als:
   - Beatmungsgerät mit einem Vernebelungs-Device oder einer Druckgas-Dosier-Aerosol-Kammer;
   - Druckgas-Dosieraerosol-Applikator
   - Container-Inhalationsanordnung mit einer Vernebelungskammer und einem Düsenvernebler oder einem Membranvernebler;
   - Inhalationsvorrichtung mit einem Druckgas-Dosieraerosol;
   - Pulverinhalator.
8. Bakteriophagen-Sensitive-Tester-Applikation
   **dadurch gekennzeichnet, dass**
   in einem Behälter Bakteriophagen, eine bakterielle Nährlösung, sowie ein Farbstoff, angeordnet sind, wobei der Farbstoff mit bakteriellen Zellwänden interagieren kann.

## Patentansprüche

1. Steriles Gel umfassend Bakteriophagen zur Verwendung bei der Behandlung oder Prophylaxe einer bakteriellen Protheseninfektion.
